# EUROPÄISCHE PATENTSCHRIFT

(11) **EP 0 645 364 B1**
(45) Veröffentlichungstag und Bekanntmachung des Hinweises auf die Patenterteilung: **20.01.1999**
(21) Anmeldenummer: 94114518.7
(22) Anmeldetag: 15.09.1994
(51) Int. Cl.: C07C 68/02, C07C 69/96

(54) **Verfahren zur Herstellung von Arylcarbonaten**
Process for the preparation of aryle carbonates
Procédé de préparation de carbonates d'aryle

(30) Priorität: 28.09.1993 DE 4332979
(43) Veröffentlichungstag der Anmeldung: 29.03.1995
(73) Patentinhaber: BAYER AG, 51368 Leverkusen (DE)
(72) Erfinder: Ooms, Pieter, Dr., D-47800 Krefeld (DE); Schön, Norbert, Dr., D-64289 Darmstadt (DE); Buysch, Hans-Josef, Dr., D-47809 Krefeld (DE)

(56) Entgegenhaltungen:
- WO-A-91/06526
- US-A- 5 239 105

## Beschreibung

Die Erfindung betrifft ein Verfahren zur Herstellung von Carbonaten mit aromatischen Estergruppen durch Umsetzung von aromatischen Monohydroxyverbindungen mit Phosgen oder Chlorkohlensäureestern aromatischer Monohydroxyverbindungen unter Abspaltung von Chlorwasserstoff in Gegenwart von einem oder mehreren Oxid(en) des Titans, Zirkoniums oder Hafniums als heterogenen Katalysatoren.

Carbonate mit aromatischen Estergruppen eignen sich zur Herstellung von Polycarbonaten nach dem Schmelzumesterungsverfahren, zur Herstellung von Phenylurethanen oder sind Vorprodukte von Wirkstoffen aus dem Pharma- und Pflanzenschutzsektor.

Es ist bekannt, daß Arylcarbonate durch Phasengrenzflächenphosgenierung (Schotten-Baumann-Reaktion) von aromatischen Hydroxyverbindungen gewonnen werden können. Dabei wirkt sich die Verwendung von Lösungsmitteln und Natronlauge nachteilig aus, da durch die wässrige Lauge eine teilweise Verseifung von Phosgen oder Chlorkohlensäureester stattfinden kann. In jedem Falle werden große Mengen Kochsalz als Nebenprodukt erhalten. Ferner muß Sorge für die Lösungsmittel-Rückgewinnung getragen werden.

Man hat daher eine Kondensation ohne Mitverwendung von Lösungsmitteln in Gegenwart von Tetramethylammoniumhalogeniden als Katalysatoren vorgeschlagen (US-A-2 837 555). Dabei sind daher die benötigten Katalysatormengen relativ groß. Man muß in der Regel mit 5 bis 7 Gew.-% Katalysator, bezogen auf die eingesetzte Phenolmenge, arbeiten, um wirtschaftliche Reaktionsgeschwindigkeiten zu erhalten; die Reaktionstemperaturen von 180°C bis 215°C bringen die Gefahr einer Zersetzung der thermolabilen Tetramethylammoniumhalogenide mit sich. Der Katalysator muß ferner anschließend durch Waschen mit Wasser entfernt werden, wodurch seine Rückgewinnung erheblich erschwert wird. Darüberhinaus wird weit mehr als die stöchiometrisch notwendige Menge an Phosgen verbraucht.

Nach einem weiteren Verfahren (US-A-3 234 263) werden Diarylcarbonate durch Erhitzen von Phenylchlorkohlensäureestern in Gegenwart großer Mengen (Erd)alkalimetallverbindungen mit tertiären Stickstoffbasen als Katalysatoren erhalten. Dieses Verfahren hat jedoch den Nachteil, daß man hohe Temperaturen anwenden und die Katalysatoren wie (Erd)Alkaliverbindungen teilweise lösen muß, um auch nur annähernd auf wirtschaftlich vertretbare Reaktionszeiten zu kommen. Bei diesem Verfahren geht die Hälfte des ursprünglich eingesetzten Phosgens in Form von CO₂ verloren. Außerdem muß man in einem vorgelagerten separaten Verfahrensschritt die Chlorkohlensäureester synthetisieren.

Nach der CA-A-2 058 359 (US-PS 167 946) erhalt man Diarylcarbonate durch Phosgenierung von aromatischen Hydroxyverbindungen in Gegenwart von Aluminiumverbindungen, die unter den Reaktionsbedingungen zumindest teilweise löslich sind, bzw. in lösliche Aluminiumhalogenide übergehen und offensichtlich auf diese Art als homogene Katalysatoren wirken (vergl. US-A-2 362 865, Sp. 1, Z. 45 bis 53). Besonders bevorzugt ist darum auch Aluminiumtrichlorid (Löslichkeit). Obwohl sehr gute Ausbeuten erhalten werden, ist es schwierig, die Katalysatoren von den Produkten zu trennen. Man muß sogar bei Destillationen mit einer gewissen Flüchtigkeit dieser Verbindungen und auch mit thermischen Zersetzungen durch diese Aluminiumverbindungen rechnen, die zu Verunreinigungen, Qualitätsminderungen und Ausbeuteeinbußen führen. Ähnliches gilt für das Verfahren der US-A-2 362 865, die noch die Verwendung von metallischem Titan, Eisen, Zink und Zinn oder in Form ihrer löslichen Salze, besonders der Chloride und Phenolate nennt.

Somit erscheint es sinnvoll, heterogene, nicht lösliche Katalysatoren zu verwenden, die eine Aufarbeitung des Reaktionsgemisches wesentlich erleichtern. Auch dazu wurden Vorschläge gemacht. So wird nach der Lehre der EP-A-516 355 vor allem Aluminiumtrifluorid empfohlen, das gegebenenfalls auf Träger wie Alumosilikate aufgebracht wird. Die Synthese von Aluminiumfluorid ist jedoch durch die Handhabung von Fluor oder Flußsäure sehr aufwendig und teurer. Weiter werden in WO 91/06526 Metallsalze auf porösen Trägern als Katalysatoren für die erfindungsgemäßen Umsetzungen beschrieben. Wie aus den Versuchsbeispielen hervorgeht, ist eine vollkontinuierliche Phosgenierung von Phenol an solchen Katalysatoren nur in der Gasphase möglich, was aber relativ hohe Reaktionstemperaturen und die Gefahr der Zersetzung der empfindlichen Chlorameisensäureester mit sich bringt. Offensichtlich ist eine Phosgenierung von Phenol mit diesen Katalysatoren in der Flüssigphase nicht durchführbar, da das heiße, flüssige Phenol die aktiven Katalysatorbestandteile auswäscht.

Die Aufgabe der Erfindung bestand also darin, einfacher zugängliche, wirksame heterogene Katalysatoren zu entwickeln.

Es wurde nun gefunden, daß Oxide des Titans, Zirkoniums oder Hafniums ausgezeichnete Katalysatoren für die Umsetzung von Phosgen oder Chlorkohlensäureestern mit aromatischen Hydroxyverbindungen darstellen. Dies ist besonders überraschend und unerwartet, weil solche Verbindungen nach der vorgängigen Lehre der WO 91/06526 als inert bekannt sind. Eine katalytische Aktivität im Sinne der vorliegenden Erfindung wird mit keinem Wort berichtet. Im Gegenteil werden Titanoxid und Zirkonoxid bevorzugt als widerstandsfähige und inerte Trägermaterialien genannt.

Der Gegenstand der vorliegenden Erfindung ist demnach ein Verfahren zur Herstellung von Arylcarbonaten durch Umsetzung von aromatischen Monohydroxyverbindungen mit Phosgen oder Chlorameisensäureestern aromatischer Monohydroxyverbindungen, das dadurch gekennzeichnet ist, daß man bei Temperaturen im Bereich von 50 bis 350°C, bei einem Druck von 0,2 bis 20 bar in Gegenwart von einem oder mehreren Oxid(en) des Titans, Zirkoniums oder Hafniums als heterogenen Katalysatoren arbeitet.

Das erfindungsgemäße Verfahren hat den großen Vorteil, daß man den Katalysator sehr leicht abtrennen kann und keine Verunreinigungen im rohen Reaktionsprodukt verbleiben. Die Aufarbeitung wird dadurch wesentlich vereinfacht.

Aromatische Monohydroxyverbindungen für das erfindungsgemäße Verfahren sind solche der Formel

Ar¹-OH (I),

worin
- Ar¹: Phenyl, Naphthyl, Anthryl, Phenanthryl, Indanyl, Tetrahydronaphthyl oder den Rest eines 5- oder 6-gliedrigen aromatischen Heterocyclus mit 1 oder 2 Heteroatomen aus der Gruppe von N, O und S bedeutet, wobei diese isocyclischen und heterocyclischen Reste durch 1 oder 2 Substituenten wie geradkettiges oder verzweigtes C₁-C₄-Alkyl, geradkettiges oder verzweigtes C₁-C₄-Alkoxy, die mit Phenyl, Cyano und Halogen (z.B. F, Cl, Br) substituiert sein können und wobei weiterhin die heterocyclischen Reste mit einem ankondensierten Benzolkern verbunden sein können.

Beispiele für aromatische Monohydroxyverbindungen der Formel (I) sind: Phenol, o-, m- und p-Kresol, o-, m- und p-Isopropylphenol, die entsprechenden Halogen-bzw. Alkoxyphenole, wie p-Chlorphenol bzw. p-Methoxyphenol, ferner Monohydroxyverbindungen des Naphthalins, Anthracens und Phenanthrens, weiterhin 4-Hydroxypyridin und Hydroxychinoline. Vorzugsweise werden gegebenenfalls substituierte Phenole, ganz besonders bevorzugt Phenol selbst eingesetzt.

Das erfindungsgemäße Verfahren kann sowohl mit Phosgen als auch mit Chlorkohlensäureestern aromatischer Monohydroxyverbindungen durchgeführt werden. Für den Fall der Durchführung mit Phosgen entsteht zunächst der Chlorkohlensäureester der mit weiterer im Reaktionsgemisch vorhandener aromatischer Monohydroxyverbindung zum Diarylcarbonat umgesetzt wird.

Geht man von Chlorkohlensäureestern und einer aromatischen Monohydroxyverbindung aus, können symmetrische oder unsymmetrische Carbonate erhalten werden.

Geeignete aromatische Chlorkohlensäureester für das erfindungsgemäße Verfahren sind demnach solche der Formel (II)

Ar¹-OCOCl (II),

worin Ar¹ die bei Formel (I) angegebene Bedeutung hat.

Die Oxide des Titans, Zirkoniums und Hafniums können in kristalliner Form in verschiedenen Modifikationen vorliegen. Sie können ganz oder teilweise amorph sein.

Solche Metalloxide und deren Herkunft bzw. Herstellverfahren für solche Verbindungen sind beispielsweise in Kirk-Othmer, Encyclopedia of Chemical Technology, 3. Auflage, Bd. 17, S. 801 ff, Bd. 23, S. 137 ff, Bd. 24, S. 882 ff, Bd. 12, S. 77 ff., New York 1978 und Ullmann's Encyclopedia of industrial chemistry, 5. Aufl.., Bd. 17 ff, S. 430, Weinheim 1985 beschrieben.

Dabei kommen sowohl Metalloxide aus natürlichen Quellen, d.h. aus verschiedenen Mineralien wie z.B. Ilmenit aber auch solche aus anderen Vorprodukten, wie beispielsweise Metallsalzen und Metallalkoxiden in Frage.

Bei den im erfindungsgemäßen Verfahren einzusetzenden Metalloxiden handelt es sich bevorzugt um Oxide des vierwertigen Titans, welche auch Oxide von Titan in niederen Oxidationsstufen enthalten können und Oxide des vierwertigen Zirkons und Hafniums, beispielsweise Titandioxid, vorkommend als Anatas, Brookit oder Rutil, Zirkondioxid in monokliner (Baddeleyit, Zirkonerde), tetragonaler, orthorhombischer oder kubischer Form und Hafniumdioxid in verschiedenen Modifikationen.

Die Metalloxide im Sinne der Erfindung können getrocknet, teilgetrocknet oder als Hydrate eingesetzt werden.

Durch sukzessive Entwässerung (Calcinierung) von Metallhydroxiden und Metalloxidhydroxiden bei Temperaturen von 80 bis oberhalb von 1200°C entstehen zunächst teilentwässerte Metalloxide, die noch merkliche Mengen an Hydroxylgruppen enthalten und mit fortschreitender Entwässerung in die wasserfreien Metalloxide übergehen. Je nach Art des Ausgangshydroxids, bzw. -oxidhydroxids können bei der Calcinierung verschiedene der obengenannten Modifikationen des Metalloxids durchlaufen werden

Die Metalloxide werden als solche verwendet, vorzugsweise die natürlich vorkommenden, können aber in untergeordnetem Maß auch andere Elemente wie Alkali- und Erdalkalimetalle, Chrom, Vanadium, Mangan, Zink, Eisen oder Silizium enthalten. Bevorzugt werden Produkte mit Gehalten an solchen Verunreinigungen von <2 Gew.-%, besonders bevorzugt <1 Gew.-% verwendet. Besonders rein sind synthetische Metalloxide. Bevorzugte Metalloxide besitzen BET-Oberflächen von 2 bis 500 m²/g, besonders bevorzugte solche von 4 bis 450 m²/g und ganz besonders bevorzugte solche von 5 bis 400 m²/g. Es können saure, neutrale und basische Oxide verwendet werden.

Die Katalysatoren können z.B. als Pulver oder Formkörper eingesetzt werden und nach der Umsetzung z.B. durch Filtration, Sedimentation oder Zentrifugieren abgetrennt werden. Für den Fall der Anordnung als Festbett werden die Metalloxide vorzugsweise als Formkörper, z.B. als Kugeln, Zylinder, Stabchen, Hohlzylinder, Ringe usw. eingesetzt.

Die Metalloxid-Katalysatoren werden beim Arbeiten mit suspendiertem Katalysator in Rührgefäßen oder Blasensäulen in Mengen von 0,5 bis 100 Gew.-%, bevorzugt von 5 bis 100 Gew.-% und besonders bevorzugt von 5 bis 50 Gew.-%, bezogen auf die eingesetzte Menge an Monohydroxyverbindung, verwendet.

Im Falle einer kontinuierlichen Fahrweise im Gegen- oder Gleichstrom oder in der Rieselphase am Festbettkatalysator werden Katalysatorbelastungen von 0,1 bis 20 g aromatische Hydroxyverbindung pro g Katalysator pro Stunde, bevorzugt 0,2 bis 10 g · g⁻¹ · h⁻¹ und besonders bevorzugt von 0,2 bis 5 g · g⁻¹ · h⁻¹ verwendet.

Die in diskontinuierlichen Versuchen verwendeten Metalloxide können bei gleichen Einsatzstoffen ohne Reinigung wiederholt eingesetzt werden. Bei einem Wechsel der Einsatzstoffe werden die Metalloxide zweckmäßig durch Extrahieren mit inerten Lösungsmitteln, wie sie beispielsweise weiter unten als Reaktionsmedien genannt sind, oder mit Alkoholen, wie Methanol, Ethanol, Isopropanol oder Butanol, mit Estern oder Amiden der Essigsäure oder durch Behandlung mit überhitztem Wasserdampf oder Luft gereinigt.

Bei kontinuierlicher Arbeitsweise können die eingesetzten Metalloxide über lange Zeit im Reaktor verbleiben. Eine Regenerierung kann gegebenenfalls durch Überleiten von überhitztem Wasserdampf gegebenenfalls unter Zugabe von untergeordneten Mengen an Luft (etwa 0,1 bis 20 Gew.-%, bezogen auf die eingesetzte Wasserdampfmenge) bei 150 bis 800°C oder durch Überleiten von 0,01 bis 20 Gew.-% Sauerstoff enthaltenden Verdünnungsgasen wie Stickstoff, oder Kohlendioxid oder durch Kohlendioxid allein bei 200 bis 800°C erfolgen. Die bevorzugte Regenerierungstemperatur liegt bei 150 bis 700°C, besonders bevorzugt bei 200 bis 600°C.

Das erfindungsgemäße Verfahren wird bei einer Temperatur im Bereich von 50 bis 350°C, bevorzugt 100 bis 300°C, besonders bevorzugt 100 bis 250°C durchgeführt. Während der Durchführung des erfindungsgemäßen Verfahrens kann die Temperatur im genannten Bereich verändert, in bevorzugter Weise erhöht werden.

Das erfindungsgemäße Verfahren wird bei einem Druck von 0,2 bis 20 bar, vorzugsweise 1 bis 5 bar, durchgeführt.

Man kann das erfindungsgemäße Verfahren unter Mitwirkung von Lösungsmitteln wie aliphatischen und aromatischen Kohlenwasserstoffen, wie Pentan, Hexan, Octan, Benzol, isomeren Xylolen, Diethylbenzol, Alkylnaphthalinen, Biphenyl; halogenierten Kohlenwasserstoffen, wie Dichlormethan, Trichlorethylen usw. durchführen.

Vorzugsweise wird das Verfahren in der Schmelze durchgeführt, beispielsweise, indem man in eine Suspension eines Metalloxides in einer Schmelze der aromatischen Monohydroxyverbindung der Formel (I) Phosgen oder einen Chlorkohlensäureester der Formel (II) einleitet und nach Beendigung der Reaktion den Katalysator z.B. durch Filtration oder Zentrifugieren abtrennt.

Eine weitere bevorzugte Ausführungsform der Synthese ist die Begasung einer Schmelze der aromatischen Monohydroxyverbindung der Formel (I) mit darin suspendiertem Metalloxid-Katalysator mit Phosgen oder Phosgen-Chlorwasserstoff-Gemischen oder mit Chlorkohlensäureestern der Formel (II) in einer kontinuierlich arbeitenden Blasensäule bzw. Blasensäulen-Kaskade.

Eine weitere bevorzugte Durchführungsform ist das Gleichstromverfahren, bei dem aromatische Hydroxyverbindungen der Formel (I) und Phosgen bzw. Chlorkohlensäureester der Formel (II) im Gleichstrom beispielsweise von oben her auf eine in einem Rohr angeordnete Katalysatorschüttung aufgebracht und unten am Fuß des Rohres Chlorwasserstoff und Phosgenierungsprodukte abgezogen werden.

Eine weitere bevorzugte Ausführungsform mit besonders günstigen Ergebnissen ist die Durchführung der erfindungsgemäßen Umsetzung in der Kieselphase, wobei die aromatische Monohydroxyverbindung der Formel (I) als Schmelze oder in Form einer Lösung oben auf ein Bett von Metalloxid gegeben wird und diesem Flüssigkeitstrom von unten ein Strom von Phosgen oder Chlorkohlensäureester entgegengeschickt wird. Zweckmäßig wird diese Ausführungsform in einem senkrecht stehenden Rohrreaktor durchgeführt, die auch Zwischenböden zur verbesserten Verteilung von Gas- und Flüssigkeitsstrom enthalten kann.

Das molare Verhältnis der Reaktionspartner aromatische Monohydroxyverbindungen der Formel (I) zu Phosgen beträgt 0,5 bis 8:1, bevorzugt 1,5 bis 3:1. Das äquivalente molare Verhältnis ist in diesem Fall 2:1.

In entsprechender Weise wird die aromatische Monohydroxyverbindung mit einem Chlorkohlensäureester im Molverhältnis von 0,25 bis 4:1, bevorzugt 0,8 bis 1,5:1 umgesetzt. In diesem Fall beträgt das molare Verhältnis 1:1.

Das durch heterogene Katalyse gewonnene rohe aromatische Carbonat ist häufig schon sehr rein und kann nach Entgasung von restlichem Chlorwasserstoff oder anderen flüchtigen Stoffen bereits in dieser Form für viele Zwecke verwendet werden. Für anspruchsvollere Anwendungen kann das Carbonat gegebenenfalls z.B. durch Destillation oder Kristallisation weiter gereinigt werden.

### Beispiele

### Beispiel 1

In einem Planschlifftopf mit Strombrechern, einem Begasungsrührer und Rückflußrührer wurden 141 g (1,50 mol) Phenol in Gegenwart von 14,1 g (10 Gew.-% bezogen auf Phenol) eines pulverförmigen Titandioxids der Firma Riedel de Haen bei 140°C mit 0,75 mol/h Phosgen kontinuierlich begast. Nach etwa 2 h Reaktionszeit betrug der Phenolumsatz 12,5 %, wobei 0,7 g Chlorameisensäurephenylester und 19,5 g Diphenylcarbonat gebildet wurde. Die Selektivität zu Kohlensäureestern war >99 %.

### Beispiel 2

Das Beispiel 1 wurde mit 14,1 g eines Titandioxid-Granulates (4 bis 5 mm Durchmesser) Calsicat 20503 der Fa. Calsicat bei 140°C wiederholt. Nach 2 h Reaktionszeit betrug der Phenolumsatz 7,2 %, wobei 0,8 g Chlorameisensäurephenylester und 11,0 g Diphenylcarbonat gebildet wurde. Die Selektivität zu Kohlensäureestern war größer als 99 %.

### Beispiel 3

Das Beispiel 1 wurde mit 14,1 g eines pulverförmigen Zirkondioxids der Fa. Aldrich bei 140°C wiederholt. Nach 2 h Reaktionszeit betrug der Phenolumsatz 6,3 %, wobei 3,2 g Chlorameisensäurephenylester und 7,8 g Diphenylcarbonat gebildet wurde. Die Selektivität zu Kohlensäureestern war größer als 99 %.

### Beispiel 4

Das Beispiel 1 wurde mit 14,1 g eines pulverförmigen Hafniumdioxids der Fa. Aldrich bei 140°C wiederholt. Nach 2 h Reaktionszeit betrug der Phenolumsatz 3,7 %, wobei 3,5 g Chlorameisensäurephenylester und 3,3 g Diphenylcarbonat gebildet wurde. Die Selektivität zu Kohlensäureestern war ca. 97 %.

### Beispiel 5 (zum Vergleich)

Das Beispiel 1 wurde ohne Zusatz von Metalloxid bei 140°C wiederholt. Nach 2 h Reaktionszeit betrug der Phenolumsatz weniger als 0,2 %.

### Beispiel 6

In einem Dreihalskolben mit Thermometer und Rückflußkühler erhitzt man ein Gemisch aus 9,4 g (0,10 mol) Phenol und 15,7 g (0,10 mol) Chlorameisensäurephenylester in Gegenwart von 0,94 g (10 Gew.-% bezogen auf Phenol) eines pulverförmigen Titandioxids der Fa. Fluka auf 100°C. Nach 5 h Reaktionszeit wird ein Phenolumsatz von 2,1 % zu Diphenylcarbonat gefunden. Die Carbonatselektivität war >99 %.

### Beispiel 7

Das Beispiel 6 wurde mit demselben Katalysator bei 120°C wiederholt. Nach 3 h Reaktionszeit betrug der Phenolumsatz zu Diphenylcarbonat 22,3 %. Die Carbonatselektivität war >99 %.

### Beispiel 8

Das Beispiel 6 wurde mit demselben Katalysator bei 140°C wiederholt. Nach 5 h Reaktionszeit betrug der Phenolumsatz zu Diphenylcarbonat 83,8 %. Die Carbonatselektivität war >99 %.

### Beispiel 9

Das Beispiel 6 wurde mit demselben Katalysator bei 160°C wiederholt. Nach 1 h Reaktionszeit betrug der Phenolumsatz zu Diphenylcarbonat 99 %. Die Carbonatselektivität war >99 %.

### Beispiel 10

Das Beispiel 6 wurde mit 0,94 g eines pulverförmigen Titandioxids (Rutil) der Fa. Bayer bei 160°C wiederholt. Nach 1 h Reaktionszeit betrug der Phenolumsatz zu Diphenylcarbonat 100 %. Die Carbonatselektivität war >99 %.

### Beispiel 11

Das Beispiel 6 wurde mit 0,94 g eines Titandioxid-Granulates (4-5 mm Durchmesser) Calsicat 20503, der Fa. Calsicat bei 160°C wiederholt. Nach 1 h Reaktionszeit betrug der Phenolumsatz zu Diphenylcarbonat 63,2 %. Die Carbonatselektivität war >99 %.

### Beispiel 12

Das Beispiel 6 wurde mit 0,94 g eines pulverförmigen Titandioxids (Anatas) der Fa. Bayer bei 160°C wiederholt. Nach 1 h Reaktionszeit betrug der Phenolumsatz zu Diphenylcarbonat 77,3 %. Die Carbonatselektivität war >99 %.

### Beispiel 13

Das Beispiel 6 wurde mit 0,94 g eines pulverförmigen Zirkondioxids der Fa. Aldrich bei 160°C wiederholt. Nach 3 h Reaktionszeit betrug der Phenolumsatz zu Diphenylcarbonat 86,5 %. Die Carbonatselektivität war >99 %.

### Beispiel 14

Das Beispiel 6 wurde mit 0,94 g eines pulverförmigen Hafniumdioxids der Fa. Aldrich bei 160°C wiederholt. Nach 5 h Reaktionszeit betrug der Phenolumsatz zu Diphenylcarbonat 63,1 %. Die Carbonatselektivität war >99 %.

## Patentansprüche

1. Verfahren zur Herstellung von Arylcarbonaten durch Umsetzung von aromatischen Monohydroxyverbindungen mit Phosgen oder Chlorkohlensäureestern aromatischer Monohydroxyverbindungen, dadurch gekennzeichnet, daß man die Umsetzung bei einer Temperatur im Bereich von 50 bis 350 °C, bei einem Druck von 0,2 bis 20 bar in Gegenwart von einem oder mehreren Oxid(en) des Titans, Zirkoniums oder Hafniums als heterogenen Katalysatoren durchführt.

2. Verfahren nach Anspruch 1, dadurch gekennzeichnet, daß als Katalysatoren ein oder mehrere Oxid(e) des Titans, Zirkoniums oder Hafniums mit nach der BET-Methode bestimmten Oberflächen von 2 bis 500 m²/g in Mengen von 0,5 bis 100 Gew.-% bezogen auf die Menge an Monohydroxyverbindung, bei nicht vollkontinuierlicher Fahrweise bzw. mit Belastungen von 0,1 bis 20 g Monohydroxyverbindung pro g Katalysator pro Stunde bei vollkontinuierlicher Fahrweise, eingesetzt werden.

## Claims

1. Process for the production of aryl carbonates by the reaction of aromatic monohydroxy compounds with phosgene or chloroformic acid esters of aromatic monohydroxy compounds, characterised in that the reaction is performed at a temperature in the range from 50 to 350°C at a pressure of 0.2 to 20 bar in the presence of one or more oxide(s) of titanium, zirconium or hafnium as heterogeneous catalysts.

2. Process according to claim 1, characterised in that the catalysts used are one or more oxide(s) of titanium, zirconium or hafnium having surface areas determined using the BET method of 2 to 500 m²/g in quantities of 0.5 to 100 wt.%, relative to the quantity of monohydroxy compound, in the case of a not completely continuous mode of operation, or at loadings of 0.1 to 20 g of monohydroxy compound per g of catalyst per hour in the case of a completely continuous mode of operation.

## Revendications

1. Procédé pour la préparation d'arylcarbonates par mise en réaction de composés monohydroxylés aromatiques avec du phosgène ou avec des esters chlorocarboniques de composés monohydroxylés aromatiques, caractérisé en ce qu'on effectue la mise en réaction à une température dans le domaine de 50 à 350°C, sous une pression de 0,2 à 20 bar, en présence d'un ou de plusieurs oxydes du titane, du zirconium ou du hafnium à titre de catalyseurs hétérogènes.

2. Procédé selon la revendication 1, caractérisé en ce qu'on met en oeuvre, à titre de catalyseurs, un ou plusieurs oxydes du titane, du zirconium ou du hafnium possédant des surfaces, déterminées conformément au procédé BET, de 2 à 500 m²/g, dans des quantités de 0,5 à 100% en poids rapportés à la quantité du composé monohydroxylé dans un mode opératoire qui ne se déroule pas complètement en continu, respectivement avec des charges de 0,1 à 20 g du composé monohydroxylé par gramme de catalyseur par heure dans un mode opératoire se déroulant complètement en continu.
